(19) Europäisches Patentamt
European Patent Office
Office européen des brevets

(11) Publication number: 0 375 299
A1

(12) **EUROPEAN PATENT APPLICATION**

(21) Application number: 89313161.5

(22) Date of filing: 15.12.89

(51) Int. Cl.5: A61K 37/64, A61K 9/06, //(A61K37/64,31:38)

Amended claim in accordance with Rule 86 (2) EPC for the following Contracting State: ES.

(30) Priority: 19.12.88 US 285932

(43) Date of publication of application: 27.06.90 Bulletin 90/26

(84) Designated Contracting States: AT BE CH DE ES FR GB IT LI LU NL SE

(71) Applicant: MERCK & CO. INC.
126, East Lincoln Avenue P.O. Box 2000
Rahway New Jersey 07065-0900(US)

(72) Inventor: Lotti, Victor J.
214 Brookside Circle
Harleysville, PA 19438(US)
Inventor: Baldwin, John J.
621 Gypsy Hill Circle
Gwynedd Valley, PA 19437(US)

(74) Representative: Hesketh, Alan, Dr. et al
European Patent Department Merck & Co., Inc. Terlings Park Eastwick Road
Harlow Essex, CM20 2QR(GB)

# (54) Combinations of angiotensin converting enzyme, and carbonic anhydrase, inhibitors for treating glaucoma.

(57) Combinations of an angiotensin converting enzyme (ACE) inhibitor and a carbonic anhydrase (CA) inhibitor are synergistically effective in reducing intraocular pressure and thereby useful in the treatment and prevention of glaucoma.

EP 0 375 299 A1

# COMBINATIONS OF ANGIOTENSIN CONVERTING ENZYME, AND CARBONIC ANHYDRASE, INHIBITORS FOR TREATING GLAUCOMA.

## SUMMARY OF THE INVENTION

This invention is concerned with the use of a combination of an ACE inhibitor and a CA inhibitor which synergistically reduces elevated intraocular pressure and is thereby useful in the treatment and prevention of glucoma.

The invention is also concerned with synergistic ophthalmological formulations comprising as active ingredients at least one ACE inhibitor and one CA inhibitor.

## BACKGROUND OF THE INVENTION

Glaucoma is an ocular disease complex associated with an elevated pressure within the eye (i.e., ocular hypertension, intraocular pressure, IOP). As a result of the elevated IOP, damage to the optic nerve head resulting in irreversible loss of visual function may ensue. Untreated, this condition may eventually lead to blindness.

Ocular hypertension, i.e., the condition of elevated intraocular pressure without optic nerve head damage or characteristic glaucomatous visual field loss, is now believed by the majority of ophthalmologists to represent the earliest phase in the onset of glaucoma.

A number of the drugs presently employed to treat glaucoma are not entirely satisfactory, particularly in the earliest course of the disease when the side effects they produce are often worse than the symptoms of the disease.

Epinephrine used as a topical solution, must be utilized cautiously in patients with high blood pressure, diabetes, hyperthyroidism and cerebral artereosclerosis due to the possibility of systemic action.

Timolol, a clinically utilized, topically applied agent for lowering intraocular pressure, must be used with caution in patients in whom beta-adrenergic blockade may be undesirable. Systemic absorption of topically administered timolol and systemic beta blockade are responsible for the contraindication of timolol therapy for glaucoma in patients with compromised pulmonary function.

Pilocarpine, a topical drug, although considered systemically harmless and quite effective, may cause considerable local difficulties. Pupil constriction may cause the eye to lose its ability to adapt from light to dark. Accommodation may become stimulated so that the patient's refraction is sometimes incorrect and vision becomes blurred. The drug itself may cause a local vasodilation and red eyes. Irritation is common.

Carbonic anhydrase inhibitors have been used systemically but they have a number of disadvantages. While effective in lowering intraocular pressure, they often cause a numbness and tingling, gastrointestinal upsets and, frequently, depression, lethargy, a loss of appetite, and general malaise.

Many of the problems associated with Systemic CA inhibitors are overcome by the use of topically effective CA inhibitors.

With the present invention there is provided a method of treating glaucoma and ocular hypertension by the topical ocular administration of a synergistic formulation comprising as active ingredients an ACE inhibitor in combination with a topically active CA inhibitor.

## DETAILED DESCRIPTION OF THE INVENTION

This invention is concerned with a novel combination of an ACE inhibitor and a CA inhibitor which is useful in a novel treatment of glaucoma and ocular hypertension associated therewith.

The ACE inhibitors useful in the novel combination of this invention are members of the class with structural formula

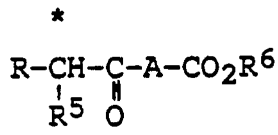

I

or a pharmaceutically acceptable salt thereof, wherein A is

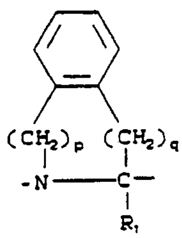

W

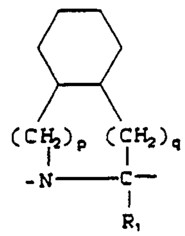

X

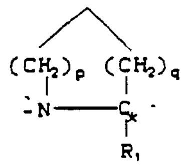

Y

or

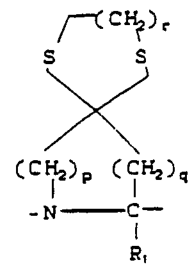

Z

;

and
R is

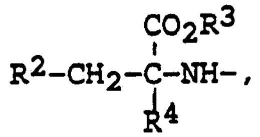

or $HS-CH_2-$
wherein:
$R_2$ is alkyl having from 1 to 6 carbon atoms, benzyl, benzylthio, benzyloxy, phenylthio or phenoxy;
$R_1$, $R_3$, $R_4$, and $R_6$ are the same or different and are hydrogen or alkyl having from 1 to 6 carbon atoms;
$R_5$ is hydrogen, alkyl having from 1 to 6 carbon atoms or aminoalkyl having from 1 to 6 carbon atoms;
p is 0, 1 or 2;
q is 0, 1 or 2, provided that the sum of p and q is 1 or 2 and that p is not 0 in formula Z;
r is 1 or 2; and the pharmaceutically acceptable salts thereof.

Preferably, in formula I, R is

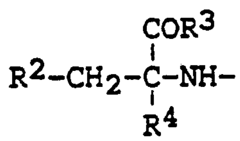

wherein $R^1$,$R^3$,$R^4$, and $R^6$ are hydrogen. It is also preferred that $R^2$ is benzyl, $R^5$ is methyl and that p, q, and r are 1. The compounds are preferably in the S,S,S,-configuration,

Preferred species for use in the novel compositions and method of treatment have the following structural formulae:

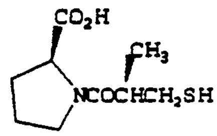

II

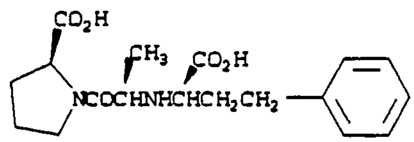

III

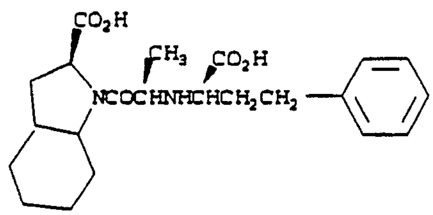

IV

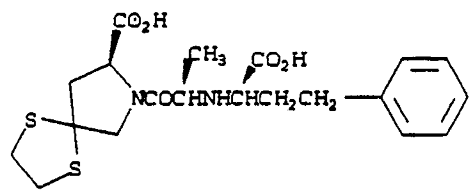

V

The CA inhibitors useful in the novel combination of this invention are aromatic or heteroaromatic sulfonamides capable of reducing elevated intraocular pressure following topical ocular administration.

Such CA inhibitors are described in U.S. Patents 4,619,939; 4,677,115; 4,668,697; U.S. Serial No. 191,085 (filed May 4, 1988); U.S. Serial No. 067,326 (filed June 26, 1987); and U.S. Serial No. 059,084 (filed June 8, 1987); the disclosures of which are incorporated herein by reference and have generic structural formula:

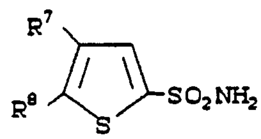

or an ophthalmologically acceptable salt thereof, wherein:

$R^7$ is hydrogen;

$R^8$ is

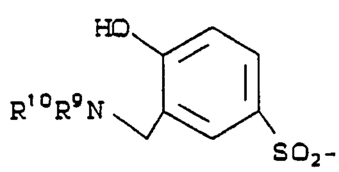 ;

or

$R^7$ and $R^8$ taken together with the carbons to which they are attached represent:

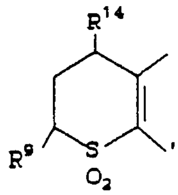,
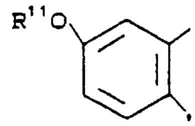, or

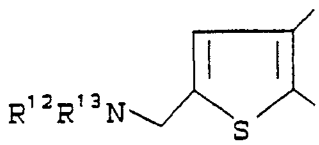

wherein $R^{14}$ is -OH or $-NHR^{10}$;
$R^{10}$ and $R^9$ are independently hydrogen or $C_{1-5}$alkyl;
$R^{11}$ is hydrogen or $C_{2-5}$alkanoyl;
$R^{13}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.

Preferred compounds within the genus have structural formulae:

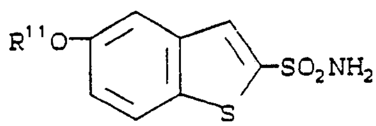

wherein $R^{11}$ is hydrogen or acetyl.

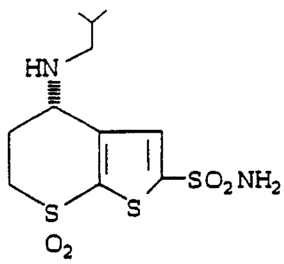,

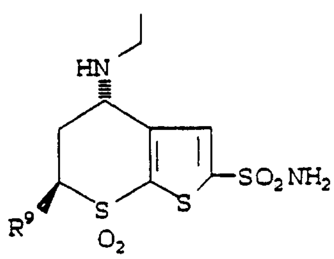

wherein $R^9$ is $-CH_3$ or $-C_2H_5$;

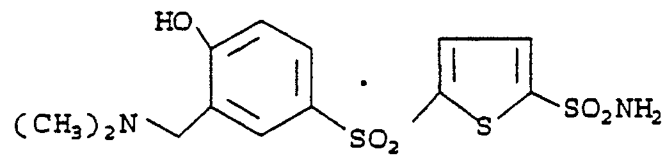 ,

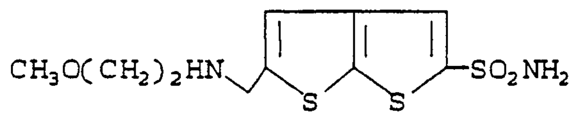

or

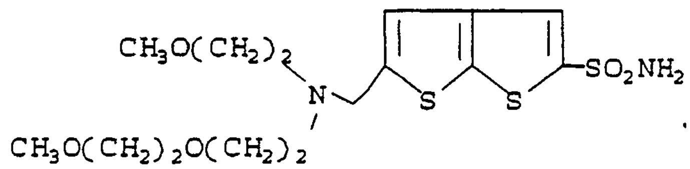 .

The combinations of ACE and CA inhibitors are administered in the form of ophthalmic pharmaceutical compositions adapted for topical administration to the eye; such as solutions, gels, suspensions, ointments and solid inserts. Each of the ACE and CA inhibitors in the ophthalmic formulation is present at a concentration of about 0.005 to 2.5% and especially about 0.125 to 1%. As a unit dosage form 0.005 to 1.25 mg., preferably 0.025 to 1.25 mg., and especially 0.05 to 0.5 mg. of each active ingredient is applied to the human eye, generally on a daily basis and preferably in divided doses.

To prepare suitable dosage forms, the active compounds may be conveniently admixed with a non-toxic pharmaceutically acceptable carrier suitable for topical ophthalmologic administration. Typical of such pharmaceutically acceptable carriers are, for example, water, mixtures of water and water miscible solvents such as lower alkanols or vegetable oils, petroleum based jelly, or including also from 0.5 to 5% by weight of water soluble polymers such as cellulose derivatives such as methyl cellulose, alkali metal carboxymethyl cellulose, hydroxyethyl cellulose, hydroxypropyl cellulose, hydroxypropyl methyl cellulose; acrylates such as polyacrylic acids salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone polyvinyl methyl ether, polyethylene oxide, neutralized carbopol and gums such as gellan, rhamson and xanthan gum and mixtures of these polymers. The pharmaceutical preparation may also contain non-toxic auxiliary substances such as emulsifying, preserving, wetting, bodying agents and the like, as for example, polyethylene glycols, carbowaxes, antibacterial preservative components commonly employed in ophthalmic formulations; buffering ingredients; and other conventional ingredients.

The pharmaceutical preparation may also be in the form of a solid insert. For example, one may use a solid water soluble polymer as the carrier for the medicament. Inserts that are known in the art that are suitable for this use include those described in British Patent 15611, and in United States Patents 3,993,071; 3,986,510; 3,868,445; and 3,867,510. Solid water insoluble inserts, such as those prepared from ethylene vinyl acetate copolymer, may also be utilized.

The compositions of the invention may include additional therapeutic agents in addition to the ACE inhibitor and CA inhibitor. For example antibiotics, autiinflammatory steroids, and anesthetics as well as other IOP lowering agents may be present.

| EXAMPLE 1 | | |
|---|---|---|
| Formulations | | |
| ACE Inhibitor | 0.5 mg. | 7.5 mg. |
| CA Inhibitor | 0.5 mg. | 7.5 mg. |
| Monobasic Sodium phosphate $\cdot 2H_2O$ | 9.38 mg. | 6.10 mg. |
| Dibasic Sodium phosphate $\cdot 12H_2O$ | 28.48 mg. | 16.80 mg. |
| Benzalkonium chloride | 0.10 mg. | 0.10 mg. |
| Water for injection q.s. ad. | 1.0 ml. | 1.0 ml. |

The active ingredient(S), phosphate buffer salts and benzalkonium chloride are added to and dissolved in water. The pH of the composition is adjusted to 5-6 and diluted to volume. The composition is rendered sterile by exposure to ionizing radiation.

## Claims

1. An ophthalmological composition for the treatment of ocular hypertension and glaucoma which comprises an ophthalmologically acceptable carrier and about 0.005 to 2.5% by weight of an ACE inhibitor and about 0.005 to 2.5% by weight of a CA inhibitor, wherein the ACE inhibitor has structural formula;

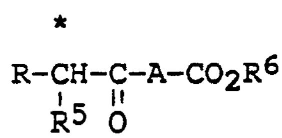

I

or a pharmaceutically acceptable salt thereof, wherein A is

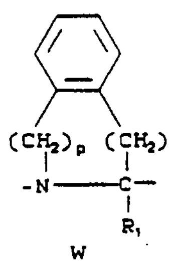

W

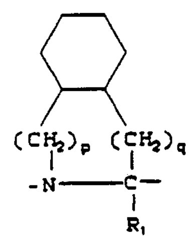

X

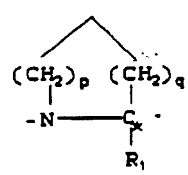

Y

or

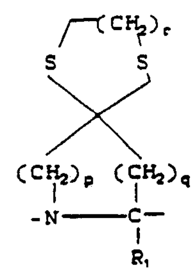

Z

;

and
R is

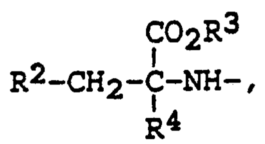

or HS-CH2-
wherein:
$R_2$ is alkyl having from 1 to 6 carbon atoms, benzyl, benzylthio, benzyloxy, phenylthio or phenoxy;

$R_1$, $R_3$, $R_4$, and $R_6$ are the same or different and are hydrogen or alkyl having from 1 to 6 carbon atoms;
$R_5$ is hydrogen, alkyl having from 1 to 6 carbon atoms or amino alkyl having from 1 to 6 carbon atoms;
p is 0, 1 or 2;
q is 0, 1 or 2, provided that the sum of p and q is 1 or 2 and that p is not 0 in formula Z;
r is 1 or 2; or a pharmaceutically acceptable salt thereof, and
the CA inhibitor has structural formula;

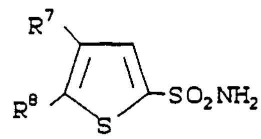

or an ophthalmologically acceptable salt thereof, wherein:
$R^7$ is hydrogen;
$R^8$ is

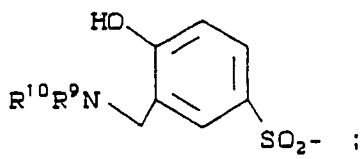

or
$R^7$ and $R^8$ taken together with the carbons to which they are attached represent:

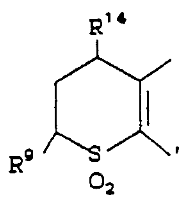

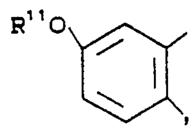 or

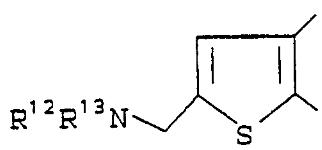

wherein $R^{14}$ is OH or $-NHR^{10}$;
$R^{10}$ and $R^9$ are independently hydrogen or $C_{1-5}$alkyl;
$R^{11}$ is hydrogen or $C_{2-5}$alkanoyl;
$R^{13}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl.

2. The formulation of Claim 1 wherein the ACE inhibitor has structural formula:

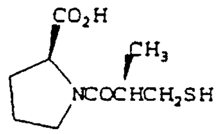

II

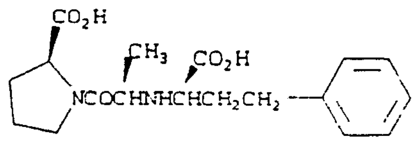

III

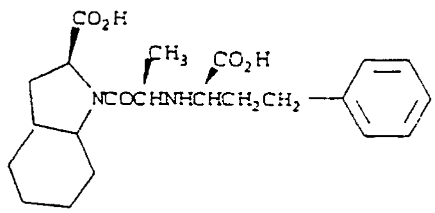

IV

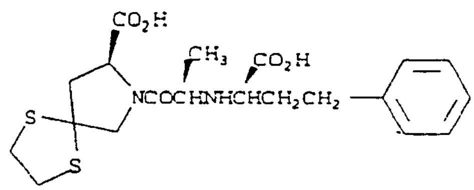

V

3. The formulation of Claim 1 or Claim 2 wherein the CA inhibitor has structural formula:

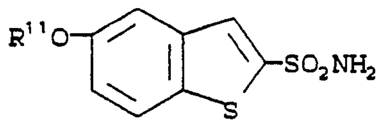

wherein $R^{11}$ is hydrogen or acetyl.

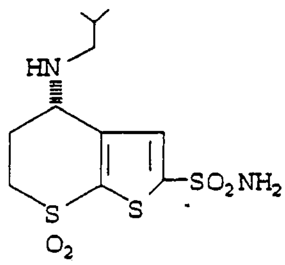

,

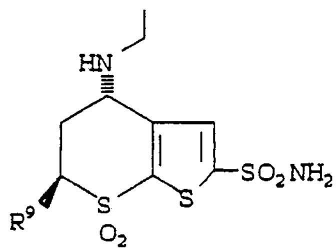

wherein $R^9$ is $-CH_3$ or $-C_2H_5$;

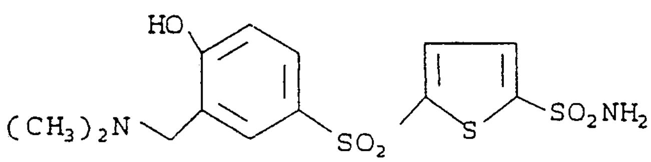

,

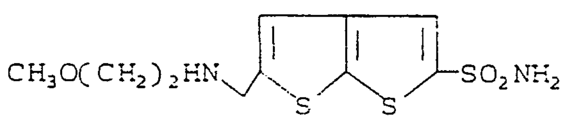

or

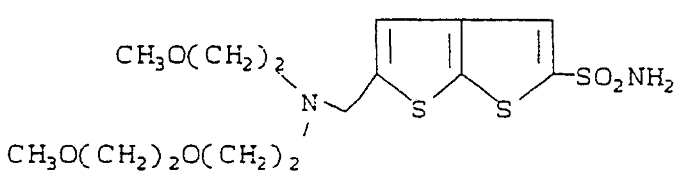

4. The use of a composition as claimed in any one of Claims 1 to 3 for the preparation of a medicament useful for treating ocular hypertension and glaucoma.

5. The use as claimed in Claim 4 wherein ACE inhibitor has structural formula:

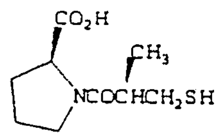

II

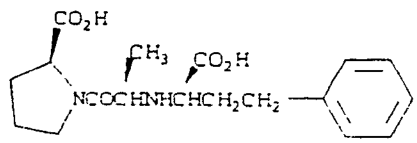

III

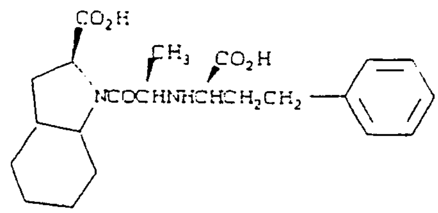

IV

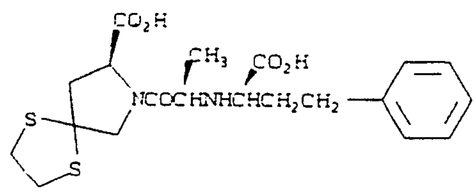

V

6. The use as claimed in Claim 4 or Claim 5 wherein the CA inhibitor has structural formula:

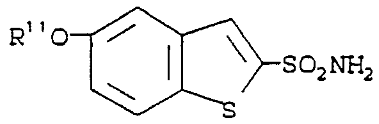

wherein $R^{11}$ is hydrogen or acetyl,

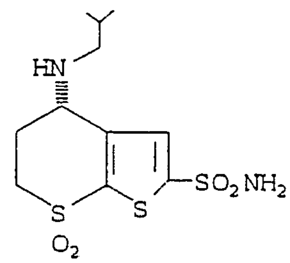

,

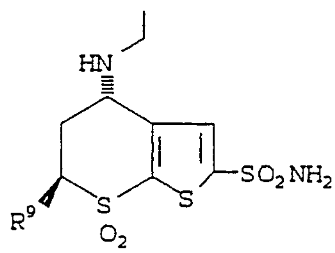

wherein $R^9$ is $-CH_3$ or $-C_2H_5$;

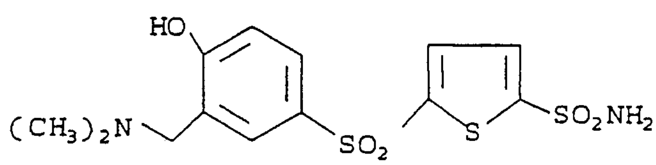

,

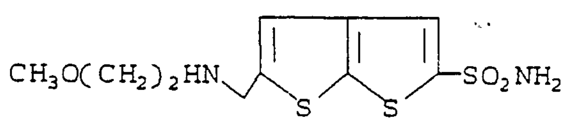

or

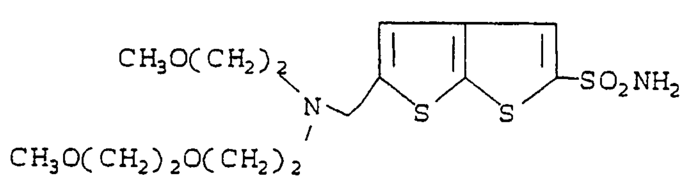

Amended claims in accordance with Rule 86(2) EPC for the following Contracting State : ES

1. A process for preparing an ophthalmological composition for the treatment of ocular hypertension

and glaucoma which comprises mixing an ophthalmologically acceptable carrier and about 0.005 to 2.5% by weight of an ACE inhibitor and about 0.005 to 2.5% by weight of A CA inhibitor, wherein the ACE inhibitor has structural formula:

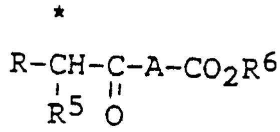

I

or a pharmaceutically acceptable salt thereof, wherein A is

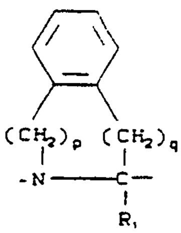
W

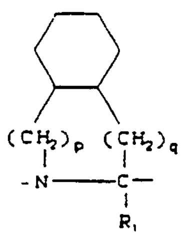
X

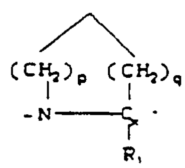
Y

or

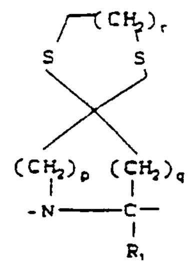
Z

;

and
R is

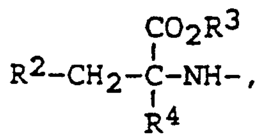

or HS-$CH_2$-
wherein:
$R_2$ is alkyl having from 1 to 6 carbon atoms, benzyl, benzylthio, benzyloxy, phenylthio or phenoxy;
$R_1$, $R_3$, $R_4$, and $R_6$ are the same or different and are hydrogen or alkyl having from 1 to 6 carbon atoms;
$R_5$ is hydrogen, alkyl having from 1 to 6 carbon atoms or amino alkyl having from 1 to 6 carbon atoms;
p is 0, 1 or 2;
q is 0, 1 or 2, provided that the sum of p and q is 1 or 2 and that p is not 0 in formula Z;
r is 1 or 2; or a pharmaceutically acceptable salt thereof, and
the CA inhibitor has structural formula;

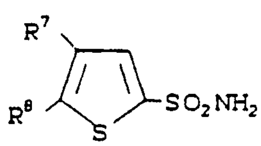

or an ophthalmologically acceptable salt thereof, wherein:
$R^7$ is hydrogen;
$R^8$ is

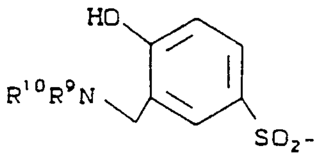
;

or
$R^7$ and $R^8$ taken together with the carbons to which they are attached represent:

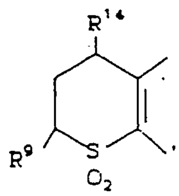
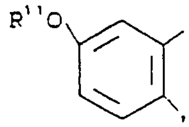
, or

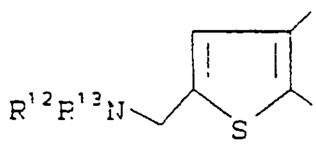

wherein $R^{14}$ is -OH or -$NHR^{10}$;
$R^{10}$ and $R^9$ are independently hydrogen or $C_{1-5}$alkyl;
$R^{11}$ is hydrogen or $C_{2-5}$alkanoyl;
$R^{13}$ is hydrogen or $C_{1-3}$alkoxy-$C_{2-4}$alkoxy-$C_{2-4}$alkyl; and
$R^{12}$ is $C_{1-3}$alkoxy-$C_{2-4}$alkyl, and optionally adding non-toxic auxiliary substances.

2. The process of Claim 1 wherein the ACE inhibitor has structural formula:

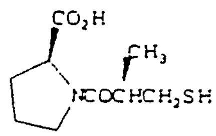

II

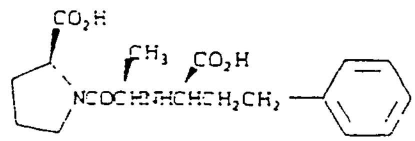

III

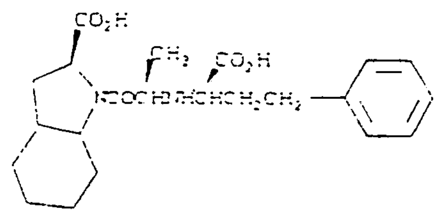

IV

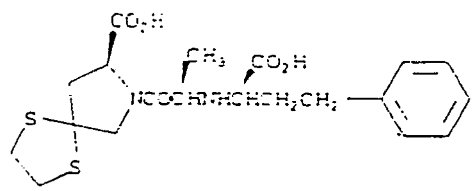

V

3. The process of Claim 1 or Claim 2 wherein the CA inhibitor has structural formula:

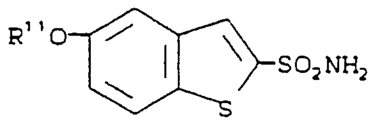

wherein $R^{11}$ is hydrogen or acetyl.

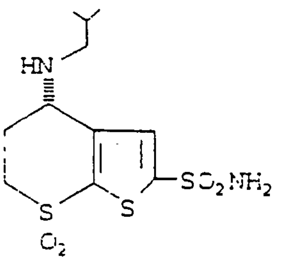

,

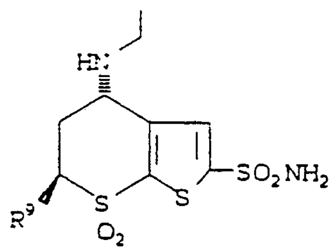

wherein $R^9$ is $-CH_3$ or $-C_2H_5$:

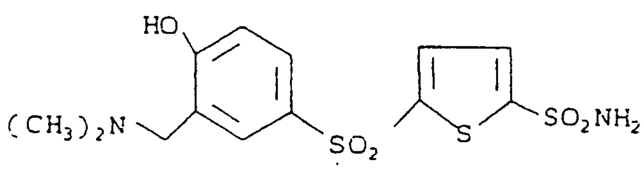

.

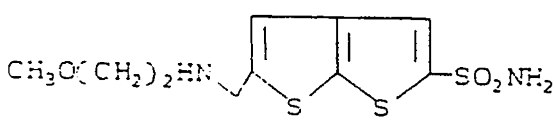

or

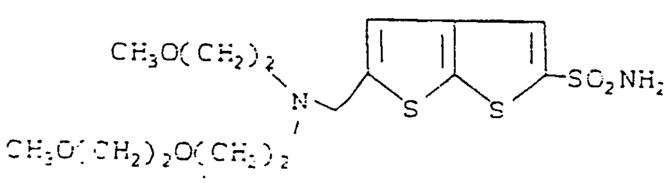

European Patent Office

# EUROPEAN SEARCH REPORT

Application Number

EP 89 31 3161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 109, no. 19, 7th November 1988, page 66, abstract no. 163473q, Columbus, Ohio, US; R.W. WATKINS et al.: "Systemic effects resulting from topical ocular administration of SCH 33861, a novel ACE inhibitor ocular hypotensive agent", & J. OCUL. PHARMACOL. 1988, 4(2), 93-100<br>* Abstract *<br>--- | 1-6 | A 61 K 37/64<br>A 61 K 9/06 //<br>(A 61 K 37/64<br>A 61 K 31:38 ) |
| Y | EP-A-0 130 109 (MERCK & CO. INC.)<br>* Pages 36-38, claims 1-6 *<br>----- | 1-6 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 06-03-1990 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)